# EUROPÄISCHE PATENTSCHRIFT

(11) **EP 2 698 135 B1**
(45) Veröffentlichungstag und Bekanntmachung des Hinweises auf die Patenterteilung: **20.07.2016**
(21) Anmeldenummer: 12005907.6
(22) Anmeldetag: 16.08.2012
(51) Int. Cl.: A61F 13/02, A61F 13/06, A61F 13/08

(54) **Elastische Binde**
Elastic bandage
Bandage élastique

(43) Veröffentlichungstag der Anmeldung: 19.02.2014
(73) Patentinhaber: Lohmann & Rauscher GmbH, 2525 Schönau/Triesting (AT)
(72) Erfinder: Steinlechner, Erik, Dr., 2500 Baden (AT); Distelrath, Iris, 56656 Brohl-Lüttzing (DE)
(74) Vertreter: Leinweber & Zimmermann

(56) Entgegenhaltungen:
- EP-A1- 1 709 947
- EP-A2- 0 878 179
- US-A- 2 687 723
- US-A1- 2008 269 654

## Beschreibung

Die Erfindung betrifft eine elastische Binde umfassend Schaum und Textilmaterial zum Erstellen eines Kompressionsverbands. Die Binde ist mindestens zweischichtig mit einer an die Haut anlegbaren, textilen inneren Schicht und einer Schaum aufweisenden äußeren Schicht.

Die elastische Binde hat also eine hautzugewandte, textile innere Schicht zur Auflage auf die Haut und eine Schaum aufweisende äußere Schicht.

Herkömmlicherweise bestehen Kompressionsverbände aus zwei Bandagen. Eine innere Bandage (Binde) wird auf die Haut des Patienten aufgelegt und dient zur Hautschonung und Polsterung. Die Binde kann bei Bedarf absorptive Eigenschaften aufweisen. Eine kohäsive äußere Bandage wird um die innere Bandage (Binde) gewickelt und dient zum Herstellen einer Kompressionswirkung auf ein therapeutisch vorgesehenes Maß, sowie zum Erhalt der Kompressionswirkung über eine Tragedauer von bis zu sieben Tagen. Zusätzlich ist die äußere Bandage zur Versteifung des Kompressionsverbands und als Widerstand gegen eine Ausdehnung der darunterliegenden Schichten vorgesehen.

Als äußere Bandagen sind imprägnierte textile Bandagen mit elastischen Fäden wie etwa gewebte Bandagen oder Vliesbandagen besonders geeignet, da mit ihnen das erforderliche Kompressionsmaß bei der Umwicklung der inneren Bandage gut einstellbar ist.

Binden bzw. innere Bandagen bestehen dagegen herkömmlicherweise aus einem an die Haut anlegbaren Schaummaterial, das sich gut an die Form des Körperteils anpasst, an dem die Binde angelegt wird, und das zusätzlich aufgrund seiner Polsterwirkung verhindert, dass die äußere Bandage lokal zu großen Druck auf den Körper des Patienten ausübt. Ferner werden exponierte Gliedmaßen wie etwa Knöchel durch die Schaumschicht geschützt. Mit anderen Worten sorgt die an der Haut anliegende Schaumschicht bei herkömmlichen Binden dafür, dass der durch die äußere Bandage ausgeübte Druck gleichmäßig auf den verbundenen Körperteil einwirkt.

In der Schrift EP 1709947 A1 ist eine Binde beschrieben, deren an die Haut anlegbare Schaumschicht an einer hautabgewandten elastischen Trägerschicht befestigt ist, wobei diese Trägerschicht mit polymerischen Bindemitteln imprägniert ist. Durch die Imprägnierung erhält die Trägerschicht kohäsive Eigenschaften, so dass die darum gewickelte äußere Bandage daran anhaftet. Solche bekannten Binden sind also zweischichtig mit einer hautzugewandten Schaumschicht und einer hautabgewandten imprägnierten Trägerschicht, auf die wiederum die äußere Bandage auflegbar ist. Jedoch hat sich herausgestellt, dass der Tragekomfort bei dieser Binde nach einiger Zeit stark nachlässt. Zusätzlich wird die auf der Haut aufliegende Schaumschicht von vielen Patienten als unangenehm empfunden.

Aus diesem Grund wurde bei bekannten Kompressionsverbänden ein so genannter Schlauchverband zwischen Haut und Schaumschicht angeordnet. Der Schlauchverband ist im Gegensatz zu der um die Extremität wickelbaren Binde eine schlauchförmige Hülse, durch die der Patient einen Arm oder ein Bein führen kann. Er besteht üblicherweise aus textilen Materialien, die vom Patienten auf der Haut als angenehm empfunden werden. Allerdings ergeben sich nach dem Anlegen eines Kompressionsverbands mit Schlauchverband oftmals Abschnürungen des verbundenen Körperteils, die darauf zurückzuführen sind, dass dieser Aufbau des Kompressionsverbands speziell für ungeübte Anwender, deren Anzahl steigend ist, sehr aufwändig, kompliziert in der Handhabung und damit stark fehleranfällig in der Anwendung ist.

Bei anderen bekannten Kompressionsverbänden, wie sie in der Druckschrift EP 0878179 A2 beschrieben sind, wird die Schaumschicht zwischen einer hautzugewandten, absorbierenden Vliesschicht und der äußeren Bandage angeordnet. Es hat sich allerdings herausgestellt, dass eine auf der Haut aufliegende Vliesschicht nachteilig für einen anhaltenden Tragekomfort ist und die gewünschte Wirkung in vielen Fällen nicht nachhaltig erreicht wird.

Die Druckschriften US 2 678 723 A und US 2008/269654 A1 offenbaren jeweils eine Binde mit einer Schaumschicht und einer an die Haut anlegbaren elastischen Gewebeschicht.

In Anbetracht der beschriebenen Probleme liegt der vorliegenden Erfindung die Aufgabe zugrunde, eine elastische Binde zum Erstellen eines Kompressionsverbands bereitzustellen, die den Tragekomfort verbessert, auch von ungeübten Anwendern einfach handhabbar, fehlerfrei anlegbar ist und über einen längeren Zeitraum beschwerdefrei und mit der gewünschten Wirkung getragen werden kann.

Diese Aufgabe wird durch eine Weiterbildung bekannter Binden gelöst, die im Wesentlichen dadurch gekennzeichnet ist, dass die an der Haut anlegbare innere Schicht der Binde ein Gewebe, Gewirk und/oder Gestrick mit synthetischen und natürlichen Fasern aufweist.

Mit anderen Worten weist die erfindungsgemäße Binde eine freiliegende textile Schicht zum Auflegen auf der Haut des Patienten auf, die in Form eines Gewebes, Gewirks und/oder Gestricks gebildet ist. Von einem Vliesstoff unterscheidet sich diese Schicht insbesondere dadurch, dass sie aus zu Garnen gesponnenen Fasern bzw. aus Fäden und nicht aus lose zusammenliegenden einzelnen Fasern besteht.

Mindestens eine hautabgewandte äußere Schicht der erfindungsgemäßen Binde weist ein Schaummaterial auf, so dass der Kompressionsdruck der um die Binde zu wickelnden äußeren Bandage gleichmäßig auf den verbundenen Körperteil einwirkt.

Die Erfindung geht auf die Erkenntnis zurück, daß sich bei der aus der EP 0 878 179 A2 bekannten Binde mit einer auf der Haut aufliegenden Vliesschicht Fasern aus der Vliesschicht herauslösen, was durch eine Wärmebildung auf der Haut nach längerem Tragen der Binde weiter gefördert wird und Juckreiz hervorruft. Andererseits lösen sich bei der erfindungsgemäßen Binde mit Gewebe, Gewirk und/oder Gestrick keine Fasern, da Fasern bei diesen Materialien in Form von gesponnenen Garnen o. dgl. verarbeitet sind, aus denen sich einzelne Fasern kaum herauslösen. Hinsichtlich der angestrebten anhaltenden Kompressionswirkung beruht die Erfindung auf der Erkenntnis, daß ein Vlies sehr rasch den - wenn auch leichten - Kompressionsdruck, der beim Anlegen des Kompressionsverbands aufgebracht wird, verliert, wodurch die anhaltende Kompressionswirkung des Kompressionsverbands stark beeinträchtigt wird. Im Gegensatz dazu wird bei erfindungsgemäßem Binden der anfänglich, im Zeitpunkt des Anlegens aufgebrachte leichte Kompressionsdruck anhaltend beibehalten.

Es hat sich herausgestellt, dass der Tragekomfort durch das auf der Haut aufliegende Gewebe, Gewirk und/oder Gestrick maßgeblich verbessert und gesteigert wird, v.a. bei mehrtägigem Tragen des Kompressionsverbands. Dies liegt insbesondere daran, dass die Oberfläche eines Schaums rau ist und eine auf der Haut aufliegende Schaumschicht aufgrund seiner Kunststoffbeschaffenheit Schweißbildung sowie aufgrund der Speicherung des Schweißes im Schaum Juckreiz verursacht. Gewebte und/oder maschige textile Stoffe werden im Gegensatz dazu auf der Haut als weich und besonders angenehm wahrgenommen. Sie verursachen kaum Schweißbildung bzw. können sich bildenden Schweiß besser aufnehmen und nach außen ableiten. Sie haben daher quasi atmungsaktive Wirkung. Der Wärmestau auf der Haut kann somit minimiert und Juckreiz kann aufgrund der Flüssigkeits- oder Dampfableitung vermindert werden. Aus diesem Grund ist die erfindungsgemäße innere Schicht der Binde besonders geeignet zur unmittelbaren Auflage auf der Haut und sogar gegebenenfalls direkt auf einer kleineren Wunde, wie z.B. auf einer oberflächlichen Schnittwunde.

Ein weiterer Vorteil der erfindungsgemäßen Binde ist deren einfache Handhabung beim Anlegen eines Kompressionsverbands. Der zu verbindende Körperteil wird in einem ersten Schritt mit der Binde umwickelt, wodurch die erste Bandagenschicht umfassend die innere Textilschicht und den hautabgewandten Schaum zu Polsterungszwecken angebracht wird. In einem zweiten Schritt wird die äußere Bandagenschicht zu Kompressionszwecken angelegt. Dies macht die erfindungsgemäße Binde auch geeignet für ungeübte Anwender, und verhindert ein fehlerhaftes Anlegen des Kompressionsverbands und Wickelfehler, die zu gefährlichen Ein- oder Abschnürungen des verbundenen Körperteils führen können. Es konnte ferner festgestellt werden, dass durch die auf die Haut zu liegen kommende Gewebe-, Gewirk- und/oder Gestrick-Schicht in Verbindung mit der äußeren Schicht umfassend Schaum eine verbesserte Stützfunktion erzielt werden kann.

Die erfindungsgemäße Binde ist nicht nur besonders gut für die Kompressionstherapie in der Phlebologie, sondern auch in der Lymphologie geeignet.

Unter einer Binde wird in der vorliegenden Beschreibung eine Materialbahn verstanden, die zu einem Wickel aufrollbar ist und eine bestimmte Länge hat, so dass sie zum Anlegen eines Verbandes um einen Körperteil eines Patienten herum wickelbar ist. Dadurch ist der Verband flexibel und an jegliche Körperkontur anpassbar.

Bevorzugte Abmessungen einer solchen Materialbahn sind in Breitenrichtung der Bahn 8 bis 12 cm und in Längenrichtung der Bahn 400 bis 800 cm.

Vorzugsweise ist die erfindungsgemäße Binde in Form eines langen und schmalen, zu einer Rolle aufgewickelten Materialbands gebildet. Bevorzugt ist die Rolle so gewickelt, dass die an der Haut anlegbare innere Schicht der Binde bei der Rolle radial nach außen zeigt. Dies erleichtert das Abwickeln der Binde um den Körperteil eines Patienten, wobei die innere, textile Schicht der Binde beim Abwickeln unmittelbar auf der Haut des Patienten zu liegen kommt. Alternativ liegt die Binde in ungewickelter Band- bzw. Streifenform vor, und hat in diesem Fall Abmessungen von vorzugsweise etwa 8 bis 12 cm Breite und etwa bis zu 1 m Länge.

Die zumindest eine äußere Schicht der Binde ist vorzugsweise wenigstens abschnittsweise durch eine Polsterschicht gebildet. Die Polsterschicht sorgt dabei für eine gleichmäßige Verteilung des von der äußeren Bandage ausgehenden Drucks und verhindert eine Abschnürung des Körperteils, an dem der Verband angebracht ist.

Die Polsterschicht kann eine Schaumschicht aufweisen. Der Schaum ist vorzugsweise ein offenporiger Polyurethan-Schaum. Durch ein Schaummaterial kann ein von außen wirkender Druck gleichmäßiger verteilt werden als durch eine Polsterschicht anderer Art wie etwa eine Watte- oder Vliesschicht. Offenporiger Polyurethan-Schaum kann Wickelfehler besonders gut ausgleichen und damit Einschnürungen vermeiden. Zweckmäßigerweise sind die Polsterschicht und die textile Schicht als etwa deckungsgleiche Flächengebilde ausgeführt.

Im Hinblick auf eine gleichmäßige Verteilung des Kompressionsdrucks der äußeren Bandage sowie im Hinblick auf eine gute Polsterwirkung hat es sich als zweckmäßig erwiesen, dass die Polsterschicht eine Dicke von etwa 0,5 mm bis etwa 5 mm, bevorzugt von etwa 1 mm bis etwa 4 mm hat.

Bei einem Gewebe als innerer Schicht der Binde haben sich folgende Kenngrößen als besonders geeignet zur Auflage auf der Haut herausgestellt: Das Gewebe sollte in ungedehntem Zustand etwa 40 bis 200, vorzugsweise 50 bis 100 Kettfäden, und/oder etwa 40 bis 200, vorzugsweise 70 bis 120, Schussfäden auf 10 cm der Gewebelänge bzw. -breite aufweisen. Diese Fadendichte ist vorteilhaft im Hinblick auf den Tragekomfort, so dass ein Kontakt der Haut mit dem Schaum vermieden wird, aber gleichzeitig hinreichend Elastizität des Gewebes gegeben ist. Alternativ oder zusätzlich kann das Gewebe aus Fäden gewebt sein, deren Fadenstärke größer als 4 tex und kleiner als 20 tex, bevorzugt größer als 6 tex und kleiner als 10 tex ist. Diese Fadenstärke ist vorteilhaft im Hinblick auf die Oberflächenglattheit und damit auf den Tragekomfort der Binde. Das Gewebe kann in Leinwand-, Atlas- und/oder Körperbindung gewebt sein, und alternativ oder zusätzlich zumindest abschnittsweise als Drehergewebe gebildet sein. Durch die Art der Webung können Dehnbarkeit und haptische sowie auch optische Eigenschaften des Gewebes gesteuert werden. Ferner hat die Webung Einfluss auf das Flächengewicht und damit auf den Tragekomfort.

Bei einer maschigen inneren Schicht der Binde haben sich folgende Kenngrößen als besonders geeignet zur Auflage auf der Haut herausgestellt: Das Gewirk oder Gestrick kann in ungedehntem Zustand etwa 100 bis 500, vorzugsweise 200 bis 400 Maschen in Längsrichtung pro 10 cm und/oder in Querrichtung aufweisen. Diese Maschendichten sind vorteilhaft im Hinblick auf Absorptionsvermögen und Dehnbarkeit des Materials. Das Gewirk oder Gestrick kann aus Fäden gebildet sein, deren Fadenstärke größer als 0,05 mm und kleiner als 0,5 mm, bevorzugt größer als 0,1 mm und kleiner als 0,3 mm ist. Diese Fadenstärken sind vorteilhaft im Hinblick auf die Oberflächenglattheit und damit auf den Tragekomfort der Binde. Je nach Wahl des Materials, beispielsweise bei der Wahl von Nylonmaterial, können die Maschenanzahl und/oder Fadenstärken auch darüber liegen. Jedenfalls ist zwecks Vermeidung des Kontakts der Haut mit dem Schaum eine weitgehend geschlossene Maschenkonstruktion besonders bevorzugt. Gestricke und Gewirke sind gegenüber Geweben aufgrund ihrer Materialstruktur dehnbarer, so dass die Elastizität der verwendeten Fäden bei Gestricken und Gewirken unter Beibehaltung einer gegebenen Materialdehnbarkeit geringer sein kann als bei Geweben. Die Binde ist demnach über die Wahl der Materialstruktur für die innere Bindenschicht hinsichtlich Haptik, Optik und Tragegefühl gut beeinflussbar sowie hinsichtlich Anpassungsfähigkeit an den mit dem Verband zu versehenden Körperteil flexibel.

Für verbesserte atmungsaktive Eigenschaften der inneren Schicht und damit einhergehend den (Ab-)Transport von Schweiß weg von der Haut weist das Gewebe, Gewirk und/oder Gestrick der erfindungsgemäßen Binde synthetische Fasern und natürliche Fasern, bspw. eine Mischung aus synthetischen und natürlichen Fasern auf. Das Gewebe, Gewirk und/oder Gestrick weist gut hautverträgliche Materialien auf, die besonders angenehm auf der Haut liegen, wie zum Beispiel Baumwolle, Viskose, Polyamid und/oder dgl. Die Verwendung von Bambus beispielsweise eignet sich besonders für eine antibakterielle Wirkung der Binde.

Ein hoher Tragekomfort der erfindungsgemäßen Binde kann dadurch sichergestellt werden, dass das Gewebe, Gewirk und/oder Gestrick ein Flächengewicht zwischen 5 g/m² und 80 g/m², bevorzugt zwischen 10 g/m² und 60 g/m², insbesondere zwischen 25 g/m² und 45 g/m² hat. Ein Material mit höherem Flächengewicht würde sich nachteilig auf die Steifheit sowie Stärke bzw. Dicke des Materials auswirken. Bei einem Material mit geringerem Flächengewicht ist ein guter Flüssigkeitsabtransport nicht mehr in der gewünschten Form gewährleistet, wodurch sich die Schweißbildung des Patienten und damit einhergehend die Entstehung von Juckreiz ungewollt erhöht.

Zum Erhalt einer erforderlichen Dehnbarkeit der Binde hat es sich als vorteilhaft erwiesen, wenn das Gewebe, Gewirk und/oder Gestrick elastische Fäden aufweist. Die Dehnbarkeit der an der Haut anlegbaren inneren Schicht kann dabei zum einen über die Materialstruktur der textilen Schicht (Art der Webung, Art der Maschen etc.), zum anderen über die Elastizität der verwendeten Garne gesteuert werden.

Das Anlegen der erfindungsgemäßen Binde ist dann besonders einfach und wenig fehleranfällig, wenn die innere Schicht mit der mindestens einen äußeren Schicht verbunden ist. Diese Verbindung kann lösbar, beispielsweise über Haftpunkte, oder alternativ unlösbar sein. Eine lösbare Verbindung hat den Vorteil, dass die äußere Schicht nach einem Austausch der inneren Textilschicht wiederverwendbar ist.

Bei einer besonders bevorzugten Ausführungsform der Erfindung liegt die Polsterschicht unmittelbar auf der textilen inneren Schicht auf und ist mit dieser verbunden. Dann können textile innere Schicht und Polsterschicht in einem einzigen Wickelschritt an dem Körperteil des Patienten angelegt werden, wodurch Zeit eingespart werden kann und Fehler beim Anlegen des Verbands vermieden werden können.

Eine unlösbare Verbindung zwischen der textilen inneren Schicht und der mindestens einen äußeren Schicht kann erfindungsgemäß mittels Flammkaschieren, Verschweißen, insbesondere Ultraschallverschweißen, erfolgen. Eine unlösbare Verbindung der beiden Schichten bereits im Verlauf der Herstellung der Binde spart Zeit und Kosten.

Die erfindungsgemäße Binde kann eine erste äußere Schicht in Form einer mit der inneren Schicht verbundenen Polsterschicht und eine außerhalb der Polsterschicht angeordnete zweite äußere Schicht haben. Die zweite äußere Schicht kann eine zweite textile Schicht sein, so dass die Polster- bzw. Schaumschicht zwischen zwei Textilschichten aufgenommen ist. Alternativ oder zusätzlich kann die zweite äußere Schicht eine kohäsive oder adhäsive hautabgewandte Oberfläche haben. Zu diesem Zweck kann die zweite äußere Schicht beschichtet oder imprägniert sein, insbesondere mit einem polymerischen Bindemittel, so dass eine äußere Bandage zur Herstellung einer Kompressionswirkung an der zweiten äußeren Schicht anhaftet. Besonders bevorzugt ist die zweite äußere Schicht eine imprägnierte Textilschicht, wie etwa eine imprägnierte Gewebeschicht.

Die Binde kann eine an die Haut anlegbare, textile innere Schicht, eine hautabgewandte, textile äußere Schicht und eine zwischen den beiden textilen Schichten angeordnete Polsterschicht aufweisen. Dabei sind die textile innere Schicht mit der Polsterschicht und die Posterschicht mit der textilen äußeren Schicht verbunden.

Die Verbindungen können lösbar oder unlösbar sein.

Die textile äußere Schicht kann eine Kompressionswirkung auf die beiden inneren Schichten und auf den mit der Binde verbundenen Körperteil entfalten. Die Notwendigkeit einer zweiten, äußeren Bandage zum Herstellen einer Kompressionswirkung kann in diesem Fall entfallen. Mit anderen Worten kann die erfindungsgemäße mindestens dreischichtige Binde als "Einlagen-Kompressionssystem" (one component compression system) verwendet werden. Das Anlegen eines solchen Einlagen-Kompressionssystems ist besonders einfach, schnell und wenig fehleranfällig, da der vollständige Kompressionsverband in einem einzigen Wickelschritt angebracht wird. Die textile innere Schicht des Einlagen-Kompressionssystems kann die bereits beschriebenen Merkmale der an der Haut anlegbaren inneren Schicht der Binde einzeln oder in Kombination miteinander aufweisen. Aus den vorstehend im Zusammenhang mit dem ersten Gesichtspunkt der Erfindung erläuterten Gründen weist die textile innere Schicht zur Auflage auf der Haut ein Gewebe, Gewirk und/oder Gestrick auf.

Die elastische Binde gemäß dem ersten oder dem zweiten Gesichtspunt der Erfindung weist vorzugsweise eine Dehnbarkeit von mehr als 20% und weniger als 100%, bevorzugt von mehr als 40% und weniger als 70% auf. Mit einer solchen Binde kann ein leichter Kompressionsdruck besonders einfach und langanhaltend erzielt werden, insbesondere aufgrund der Verwendung von Gewebe, Gewirk und/oder Gestrick. Je nach Art der Anwendung kann die Notwendigkeit einer zweiten äußeren Kompressionsbandage entfallen.

Bei einer besonders bevorzugten Ausführungsform der Erfindung ist das Dehnungsverhalten der Binde kurzzügig. Mit anderen Worten ist die Binde eine Kurzzugbinde. Mit Kurzzugsbinden kann ein bspw. bei Venenerkrankungen erwünschter hohen Arbeitsdruck und niedriger Ruhedruck erzeugt werden. Kurzzugbinden werden auch bspw. zum Verbinden von offenen Beinen wirksam eingesetzt.

Es lassen sich Kosten dadurch einsparen, dass die erfindungsgemäße elastische Binde mehrfach verwendbar ist. Aus diesem Grund ist die Binde zweckmäßigerweise waschbar, vorzugsweise waschbar bis zu einer Temperatur von 95°.

Je nach Art der Anwendung bzw. je nach Art der Wunde und des Körperteils, der mit der Binde verbunden wird, kann eine der Schichten hautpflegende, hautverbessernde, hauttemperaturregulierende, antimikrobielle, antiseptische und/oder geruchshemmende Stoffe aufweisen. Bspw. kann die textile innere Schicht der Binde einen antimikrobiellen und/oder einen antiseptischen Stoff enthalten, wenn sie zum Verbinden von offenen Beinen verwendet werden soll. Alternativ kann die textile innere Schicht der Binde einen hautpflegenden und/oder einen geruchshemmenden Stoff aufweisen, wenn die Binde bei einer Gewebeschwellung oder einem Ödem verwendet werden soll. Alternativ oder zusätzlich kann mindestens eine äußere Schicht wie etwa eine Polsterschicht und/oder Schaumschicht einen solchen Stoff enthalten. Dies hat den Vorteil, dass der Stoff dann langsamer und über einen längeren Zeitraum an die Haut abgegeben werden kann. Vorzugsweise weist aber die hautzugewandte innere Schicht mindestens einen oder mehrere solche Stoffe auf.

Die Erfindung betrifft ferner einen Zweilagen-Kompressionsverband mit einer äußeren, kohäsiven Bandage zum Erzeugen einer Kompressionswirkung und einer hautzugewandten inneren Bandage in Form einer elastischen Binde. Die Binde hat eine an die Haut anlegbare, textile innere Schicht und eine Schaum aufweisende äußere Schicht, wobei die innere Schicht ein Gewebe, Gewirk und/oder Gestrick aufweist.

Die Binde kann eine zweite äußere Schicht haben, so dass die Schaum aufweisende Schicht zwischen der textilen inneren Schicht und der zweiten äußeren Schicht angeordnet ist.

Des Weiteren betrifft die Erfindung einen Multilagen-Kompressionsverband mit mindestens einer äußeren Bandage zum Erzeugen einer Kompressionswirkung und einer hautzugewandten inneren Bandage in Form einer oben beschriebenen elastischen Binde.

In der folgenden Beschreibung wird die Erfindung unter Bezugnahme auf die Zeichnung beispielhaft erläutert. In der Zeichnung zeigt:
Fig. 1: eine schematische Darstellung einer elastischen Binde gemäß der Erfindung in deren Anwendung bei der Erstellung eines Kompressionsverbands an einen Körperteil und
Fig. 2: eine Explosionsdarstellung eines Kompressionsverbands gemäß einem weiteren Aspekt der Erfindung.

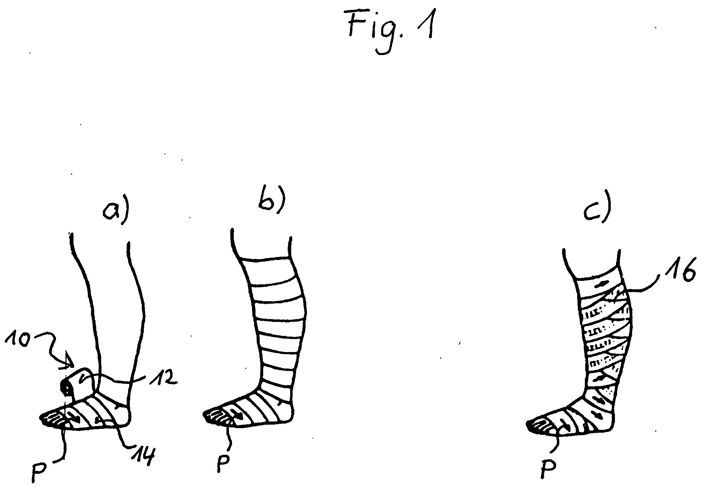

In Fig. 1 ist das Anlegen einer erfindungsgemäßen elastischen Binde zur Erstellung eines Zweilagenkompressionsverbands an die untere Extremität eines Patienten schematisch dargestellt. Gemäß Fig. 1 a wird eine Binde 10 in Form eines zu einer Rolle aufgewickelten Materialbandes eingesetzt. Die Binde weist eine in der Rolle radial nach außen weisende textilen Schicht 12 in Form eines Gewebes, Gewirks und/oder Gestricks sowie ein im angelegten Zustand auf der der Haut des Patienten abgewandten Außenseite der textilen Schicht 12 angeordneten Polsterschicht 14 auf. Die Polsterschicht 14 kann deckungsgleich zur textilen Schicht 12 ausgeführt sein und einen offenporigen Polyurethanschaum aufweisen. In Fig. 1b ist der Zustand des Patienten nach Anlegen der Binde dargestellt. Durch vergleichende Betrachtung der Fig. 1 a und 1 b wird deutlich, daß die Binde bei Behandlung von Fuß- und Unterschenkel zweckmäßigerweise anfangend von den Zehen zunächst um den Fuß, dann um das Sprunggelenk und schließlich um den Unterschenkel gewickelt wird. Nach Anlage der Binde gemäß Fig. 1b wird die zum Aufbau des gewünschten Kompressionsdrucks eingesetzte äußere Bandage angelegt. Auch die äußere Bandage 16 kann in Form eines Wickels aus einer langen und schmalen Materialbahn vorliegen. Sie wird zweckmäßigerweise in derselben Wickelrichtung wie die Binde 10 um die untere Extremität des Patienten gewickelt wie durch die Pfeile P in den Fig. 1 a und 1 c dargestellt.

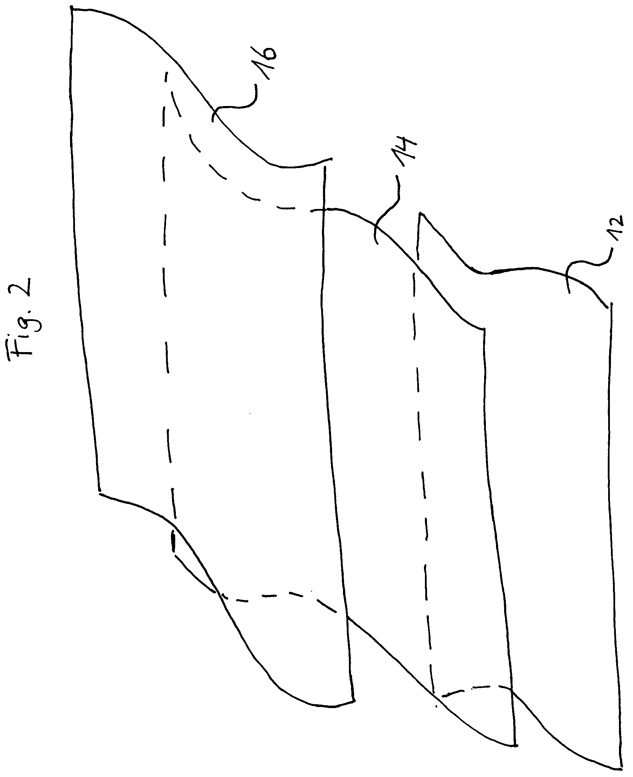

In Fig. 2 ist ein erfindungsgemäßer Kompressionsverband dargestellt. Der Kompressionsverband weist ein textiles, an die Haut anlegbares Flächengebilde 12 in Form eines Gewebes, Gewirks und/oder Gestricks auf, sowie eine Polsterschicht 14 und eine kohäsive Bandage 16. Die textile Schicht 12 und die Polsterschicht 14 können durch Klebepunkte, mittels Ultraschallschweißen oder auch mittels Flammkaschieren miteinander verbunden sein. Die kohäsive Bandage dient zum Aufbringen des gewünschten Kompressionsdrucks. Die der Polsterschicht 14 zugewandte Begrenzungsfläche der kohäsiven Bandage ist zweckmäßigerweise so ausgestattet, daß sie an der textilen Schicht 12 abgewandten Begrenzungsfläche der Polsterschicht 14 gut haftet.

Die Erfindung ist nicht auf das anhand der Zeichnungen erläuterte Ausführungsbeispiel beschränkt. Beispielsweise ist im Rahmen der Erfindung daran gedacht, die textile Schicht, die Polsterschicht und die kohäsive Bandage zu einem gemeinsamen Wickel aufzuwickeln, um so das Anlegen der Bandage weiter zu vereinfachen. Bei den anhand der Zeichnungen erläuterten Ausführungsbeispielen besteht die Polsterschicht aus einem offenporigen Polyurethanschaum. Sie kann im Rahmen der Erfindung aber auch aus zwei, drei oder mehr Komponenten bestehen. Das kann zum Aufbau eines gleichmäßigen Kompressionsdrucks vorteilhaft sein. Ferner ist daran gedacht, einen Kompressionsverband aus vier, fünf oder mehr Schichten herzustellen.

## Patentansprüche

1. Elastische Binde zum Erstellen eines Kompressionsverbands umfassend Schaum und Textilmaterial mit einer an die Haut anlegbaren, textilen inneren Schicht und mindestens einer äußeren Schicht, **dadurch gekennzeichnet, dass** die innere Schicht ein Gewebe, Gewirk und/oder Gestrick mit synthetischen Fasern wie etwa Polyamid und natürlichen Fasern wie etwa Baumwolle aufweist.

2. Binde nach Anspruch 1, **dadurch gekennzeichnet, dass** die mindestens eine äußere Schicht zumindest abschnittsweise durch eine Polsterschicht gebildet ist.

3. Binde nach Anspruch 2, **dadurch gekennzeichnet, dass** die Polsterschicht eine Schaumschicht, vorzugsweise umfassend einen offenporigen Polyurethan-Schaum, aufweist.

4. Binde nach Anspruch 2 oder 3, **dadurch gekennzeichnet, dass** die Polsterschicht eine Dicke von etwa 0,5 mm bis etwa 5 mm, bevorzugt von etwa 1 mm bis etwa 4 mm hat.

5. Binde nach einem der vorhergehenden Ansprüche, **dadurch gekennzeichnet, dass** das Gewebe, Gewirk und/oder Gestrick ein Flächengewicht zwischen 5 g/m² und 80 g/m², bevorzugt zwischen 10 g/m² und 60 g/m², insbesondere zwischen 25 g/m² und 45 g/m² hat.

6. Binde nach einem der vorhergehenden Ansprüche, **dadurch gekennzeichnet, dass** das Gewebe, Gewirk und/oder Gestrick elastische Fäden aufweist.

7. Binde nach einem der vorhergehenden Ansprüche, **dadurch gekennzeichnet, dass** die innere Schicht mit der mindestens einen äußeren Schicht lösbar, bevorzugt über Haftpunkte, verbunden ist.

8. Binde nach einem der Ansprüche 1 bis 6, **dadurch gekennzeichnet, dass** die innere Schicht mit der mindestens einen äußeren Schicht mittels Flammkaschieren oder Verschweißen, insbesondere Ultraschallverschweißen, bevorzugt unlösbar verbunden ist.

9. Binde nach einem der vorhergehenden Ansprüche, **gekennzeichnet durch** eine erste äußere Schicht in Form einer mit der inneren Schicht verbundenen Polsterschicht und eine außerhalb der ersten äußeren Schicht angeordnete zweite äußere Schicht.

10. Binde nach einem der vorhergehenden Ansprüche, **gekennzeichnet durch** eine hautabgewandte, textile äußere Schicht und eine zwischen den beiden textilen Schichten angeordnete, Schaum aufweisende Polsterschicht, wobei die textile innere Schicht mit der Polsterschicht und die Polsterschicht mit der textilen äußeren Schicht verbunden ist.

11. Binde nach einem der vorhergehenden Ansprüche, **gekennzeichnet durch** eine Dehnbarkeit von mehr als 20% und weniger als 100%, bevorzugt von mehr als 40% und weniger als 70%.

12. Binde nach einem der vorhergehenden Ansprüche, **dadurch gekennzeichnet, dass** das Dehnungsverhalten der Binde kurzzügig ist.

13. Binde nach einem der vorhergehenden Ansprüche, **gekennzeichnet durch** eine Waschbarkeit bis zu 95°.

14. Binde nach einem der vorhergehenden Ansprüche, **dadurch gekennzeichnet, dass** eine der Schichten hautpflegende, hautverbessernde, hauttemperaturregulierende, antimikrobielle, antiseptische und/oder geruchshemmende Stoffe aufweist.

15. Zweilagen-Kompressionsverband mit einer äußeren, kohäsiven Bandage zum Erzeugen einer Kompressionswirkung und einer hautzugewandten inneren Bandage in Form einer Binde nach einem der Ansprüche 1 bis 14.

16. Multilagen-Kompressionsverband mit mindestens einer äußeren Bandage zum Erzeugen einer Kompressionswirkung und einer hautzugewandten inneren Bandage in Form einer Binde nach einem der Ansprüche 1 bis 14.

## Claims

1. An elastic bandage for creating a compression dressing comprising foam and textile material having a textile inner layer that can be placed against the skin and at least one outer layer, **characterised in that** the inner layer has a woven, mesh and/or knit fabric with synthetic fibres such as for example polyamide and natural fibres such as for example cotton.

2. The bandage according to Claim 1, **characterised in that** the at least one outer layer is formed at least partially by a cushioning layer.

3. The bandage according to Claim 2, **characterised in that** the cushioning layer has a foam layer, preferably comprising an open-cell polyurethane foam.

4. The bandage according to Claim 2 or 3, **characterised in that** the cushioning layer has a thickness of approximately 0.5 mm to approximately 5 mm, preferably from approximately 1 mm to approximately 4 mm.

5. The bandage according to any of the preceding claims, **characterised in that** the woven, mesh and/or knit fabric has a grammage between 5 g/m² and 80 g/m², preferably between 10 g/m² and 60 g/m², in particular between 25 g/m² and 45 g/m².

6. The bandage according to any of the preceding claims, **characterised in that** the woven, mesh and/or knit fabric contains elastic threads.

7. The bandage according to any of the preceding claims, **characterised in that** the inner layer is releaseably connected to the at least one outer layer, preferably via adhesive points.

8. The bandage according to any of Claims 1 to 6, **characterised in that** the inner layer is non-releaseably connected to the at least one outer layer by means of flame bonding or welding, in particular ultrasonic welding.

9. The bandage according to any of the preceding claims, **characterised by** a first outer layer in the form of a cushioning layer connected to the inner layer and a second outer layer arranged outside of the first outer layer.

10. The bandage according to any of the preceding claims, **characterised by** a textile outer layer facing away from the skin and a cushioning layer containing foam arranged between the two textile layers, the textile inner layer being connected to the cushioning layer and the cushioning layer being connected to the textile outer layer.

11. The bandage according to any of the preceding claims, **characterised by** a stretchability of more than 20% and less than 100%, preferably more than 40% and less than 70%.

12. The bandage according to any of the preceding claims, **characterised in that** the stretching response of the bandage involves short traction.

13. The bandage according to any of the preceding claims, **characterised by** washability of up to 95°C.

14. The bandage according to any of the preceding claims, **characterised in that** one of the layers contains skin-care, skin-enhancing, skin temperature-regulating, antimicrobial, antiseptic and/or odour-inhibiting substances.

15. A two-layer compression dressing having an outer cohesive bandage for generating a compression effect and an inner bandage that faces the skin in the form of a bandage according to any of Claims 1 to 14.

16. A multi-layer compression dressing having at least one outer bandage for generating a compression effect and an inner bandage that faces the skin in the form of a bandage according to any of Claims 1 to 14.

## Revendications

1. Bande élastique pour la réalisation d'un bandage compressif comprenant de la mousse et un matériau textile présentant une couche intérieure textile destinée à être appliquée contre la peau et au moins une couche extérieure, **caractérisée en ce que** la couche intérieure présente un tissu, treillis et/ou tricot de fibres synthétiques, par exemple en polyamide, et de fibres naturelles, par exemple en coton.

2. Bande selon la revendication 1, **caractérisée en ce que** l'au moins une couche extérieure est constituée, au moins par sections, d'une couche rembourrée.

3. Bande selon la revendication 2, **caractérisée en ce que** la couche rembourrée présente une couche de mousse, comprenant de préférence une mousse de polyuréthane à cellules ouvertes.

4. Bande selon la revendication 2 ou 3, **caractérisée en ce que** la couche rembourrée présente une épaisseur d'environ 0,5 mm à environ 5 mm, de préférence d'environ 1 mm à environ 4 mm.

5. Bande selon l'une quelconque des revendications précédentes, **caractérisée en ce que** le tissu, treillis et/ou tricot présente un grammage compris entre 5 g/m² et 80 g/m², de préférence entre 10 g/m² et 60 g/m², notamment entre 25 g/m² et 45 g/m².

6. Bande selon l'une quelconque des revendications précédentes, **caractérisée en ce que** le tissu, treillis et/ou tricot présente des fibres élastiques.

7. Bande selon l'une quelconque des revendications précédentes, **caractérisée en ce que** la couche intérieure est reliée à l'au moins une couche extérieure de manière amovible, de préférence par points d'adhésion.

8. Bande selon l'une quelconque des revendications 1 à 6, **caractérisée en ce que** la couche intérieure est reliée à l'au moins une couche extérieure de préférence de manière non amovible, par contrecollage à la flamme ou soudage, notamment soudage par ultrasons.

9. Bande selon l'une quelconque des revendications précédentes, **caractérisée par** une première couche extérieure sous la forme d'une couche rembourrée liée à la couche intérieure et par une seconde couche extérieure disposée sur l'extérieur de la première couche extérieure.

10. Bande selon l'une quelconque des revendications précédentes, **caractérisée par** une couche extérieure textile détournée du contact avec la peau, et par une couche rembourrée présentant de la mousse et disposée entre les deux couches textiles, la couche intérieure textile étant reliée à la couche rembourrée, et la couche rembourrée étant reliée à la couche extérieure textile.

11. Bande selon l'une quelconque des revendications précédentes, **caractérisée par** une extensibilité de plus de 20 % et de moins de 100 %, de préférence de plus de 40 % et de moins de 70 %.

12. Bande selon l'une quelconque des revendications précédentes, **caractérisée en ce que** le comportement à l'allongement de la bande est à courte élasticité.

13. Bande selon l'une quelconque des revendications précédentes, **caractérisée par** une aptitude au lavage jusqu'à une température de 95°.

14. Bande selon l'une quelconque des revendications précédentes, **caractérisée en ce que** l'une des couches présente des composants de soin de la peau, de renforcement de la peau, de régulation de la température de la peau, antimicrobiens, antiseptiques et/ou inhibiteurs d'odeurs.

15. Bandage compressif à deux couches, comportant un bandage extérieur cohésif destiné à la réalisation d'un effet compressif, et un bandage intérieur en contact avec la peau sous la forme d'une bande selon l'une quelconque des revendications 1 à 14.

16. Bandage compressif multicouche comportant au moins un bandage extérieur destiné à la réalisation d'un effet compressif et un bandage intérieur en contact avec la peau sous la forme d'une bande selon l'une quelconque des revendications 1 à 14.
